# EUROPÄISCHE PATENTANMELDUNG

(11) **EP 1 749 519 A1**
(43) Veröffentlichungstag der Anmeldung: **07.02.2007**
(21) Anmeldenummer: 05090228.7
(22) Anmeldetag: 05.08.2005
(51) Int. Cl.: A61K 9/50, A61K 31/495

(54) **Arzneiform mit Retardtierter pH-unabhängiger Wirkstofffreisetzung für Wirkstoffe mit starker pH-abhängiger Löslichkeit**

(71) Anmelder: Schering Aktiengesellschaft, 13353 Berlin (DE)
(72) Erfinder: Guthmann, Claudia, 10247 Berlin (DE); Kranz, Heiko Dr., 10715 Berlin (DE); Lipp, Ralph Dr., Zionsville, IN, 46077 (US); Wagner, Torsten Dr., 13127 Berlin (DE)

(57) **Zusammenfassung**

Feste Arzneimittelformulierung für eine retardierte pH-unabhängige Wirkstofffreisetzung enthaltend mindestens eine Schicht aus einem oder mehreren wasserunlöslichen Polymeren mindestens eine Schicht aus einem oder mehreren pH-abhängig wasserlöslichen Polymeren und einem wirkstoffhaltigen Kern, mit starker pH-abhängiger Wasserlöslichkeit und mindestens einem Osmagenten enthält.

## Beschreibung

### Technisches Gebiet

Die Erfindung betrifft eine feste Arzneimittelformulierung für eine retardierte pH-unabhängige Wirkstofffreisetzung enthaltend mindestens eine Schicht aus einem oder mehreren wasserunlöslichen Polymeren, mindestens eine Schicht aus einem oder mehreren pH-abhängigen wasserlöslichen Polymeren und einen wirkstoffhaltigen Kern, wobei der Kern einen Wirkstoff mit starker pH-abhängiger Wasserlöslichkeit und mindestens einen osmotisch aktiven Stoff enthält.

Wirkstoffe mit starker pH-abhängiger Wasserlöslichkeit sind zum Beispiel Substanzen, die bei basischen pH-Werten sehr schlecht löslich sind, üblicherweise mit einer Löslichkeit im Wasser niedriger als 0,1 mg/ml, während bei sauren pH-Werten (pH < 4) die Löslichkeit bis zu Werten von 1 mg/ml oder höher geht.

Allgemein pH-abhängige wasserlösliche Wirkstoffe können auch definiert sein als Substanzen mit einem Unterschied des mindestens 10 fachen der Wasserlöslichkeit bei sauren und basischen pH-Werten.

Ein Beispiel von eines Wirkstoffs mit starker pH-abhängiger Löslichkeit im Wasser ist (2R)-1-((4-chloro-2-(ureido)phenoxy)methyl)carbonyl-2-methyl-4-(4-fluorobenzyl)piperazin oder ein Salz hiervon.

(2R)-1-((4-chloro-2-(ureido)phenoxy)methyl)carbonyl-2-methyl-4-(4-fluorobenzyl)piperazin wird im folgenden Piperazinharnstoff genannt und hat die folgende Struktur:

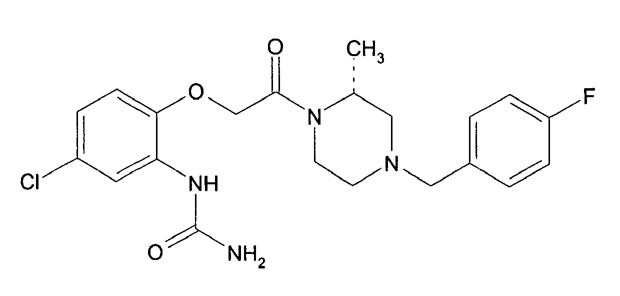

Die Herstellung von (2R)-1-((4-chloro-2-(ureido)phenoxy)methyl)carbonyl-2-methyl-4-(4-fluorobenzyl)piperazin und seinen Salzen erfolgt nach der Methode, die in WO98/56771 in Beispiel 2 beschrieben ist.

Salze hiervon sind z.B. das Hydrochlorid, Dihydrogenphosphat, Hydrogensulfat, Sulfat, Mesylat, Ethylsulfonat, Malat, Fumarat und Tartrat.

Die folgende Erfindung betrifft weiter ein Matrixpellet für eine retardierte pH-unabhängige Wirkstofffreisetzung enthaltend mindestens eine Schicht aus einem oder mehreren wasserunlöslichen Polymeren, in denen porenbildende Substanzen enthalten sind, welche nach Kontakt mit dem wässrigen Medium herausgelöst werden und dadurch eine mikroporöse Membran bilden, mindestens eine Schicht aus einem oder mehreren pH-abhängig wasserlöslichen Polymeren, und einen wirkstoffhaltigen Kern, wobei der Kern Piperazinharnstoff und mindestens eine wasserlösliche ionische Substanz aus der Gruppe von Magnesiumchlorid, Magnesiumsulfat, Lithiumchlorid, Natriumchlorid, Kaliumchlorid, Lithiumsulfat, Natriumsulfat, Kaliumsulfat, Lithiumphosphat, Natriumphosphat, Kaliumphosphat, Ammoniumchlorid, Ammoniumsulfat, Ammoniumphosphat als Osmagent enthält.

Weitere feste Arzneimittelformulierungen im Sinne der Erfindung sind single-unit Systeme, wie z.B. Tabletten, und multipartikuläre Systeme. Multipartikuläre Systeme können z.B. Granulatkörner, Pellets oder Minitabletten sein. Diese können in Hart- oder Weichgelatinekapseln gefüllt, sowie zu Tabletten verpreßt werden. Der ursprüngliche Formulierung zerfällt meist im Magen in viele Untereinheiten. Die Minidepots treten dann sukzessive aus dem Magen in den Darm über. Die Minidepots können dabei in der Regel den Pylorus bei geschlossenem Sphinkter passieren.

Retardformulierungen sind oral verabreichbare Medikamente mit einer länger anhaltenden Wirkung des Medikamentes. Dabei wird der pharmazeutische Wirkstoff verlangsamt abgegeben.

### Stand der Technik

In der Literatur sind verschiedene pharmazeutische Formulierungen für kontrollierte Wirkstoffreisetzung vorhanden.

Eine Elementare Osmotische Pumpe (EOP), zum Beispiel, sind Tabletten, welche aus einem osmotisch wirksamen Tablettenkern bestehen, der mit einer semipermeablen Membran überzogen ist, welche ein Freisetzungsloch enthält.

Der Tablettenkern kann einen osmotisch wirksamen Arzneistoff enthalten oder im Falle eines wenig osmotisch aktiven Arzneistoffes osmotisch aktive Zusätze, auch als Osmagenten allgemein definiert. Strömt Wasser durch die semipermeable Membran (SPM) in die Arzneiformen, entsteht ein hydrostatischer Druck, der den gelösten Arzneistoff durch die Freisetzungsöffnung drückt.

Aufgabe einer EOP ist die kontrollierte Wirkstofffreisetzung, wobei eine Freisetzungskinetik 0. Ordnung erreicht wird. Es wird also pro Zeiteinheit eine gleichbleibende Menge Arzneistoff aus der Arzneiform freigesetzt.

Voraussetzung für eine EOP ist ein moderat wasserlöslicher Wirkstoff.

Um auch schwer lösliche Arzneistoffe kontrolliert freisetzen zu können, wurden Push und Pull Osmotische Pumpen (PPOPs) etabliert.

Hierbei handelt es sich um Mehrkammertablettensysteme, deren Kern ein osmotisches Wirkstoffkompartiment und ein quellbares osmotisch aktives Polymer enthält, dabei werden beide Kompartimente durch ein elastisches Diaphragma getrennt. Der gesamte Tablettenkern ist wiederum durch eine SPM umhüllt, die ein Freisetzungsloch auf der wirkstoffstoffhaltigen Seite enthält.

Wasser dringt in beide Kompartimente ein, wobei das Polymer quillt und dabei das Diaphragma in das Wirkstoffkompartiment drückt. Der Wirkstoff wird nun durch die Freisetzungsöffnung abgegeben. Auch hier ist das Ziel die Schaffung gleichbleibender Plasmaspiegel durch die Wirkstofffreisetzung 0. Ordnung.

Also, Osmotisch wirkende Systeme, wie die Elementare Osmotische Pumpe (EOP) und Push und Pull Osmotische Pumpen (PPOP) setzen mindestens moderat wasserlösliche Wirkstoffe aus Tabletten frei, die aus einer semipermeablen Membran um einen osmotisch wirksamen Kern bestehen, der zumindest einer osmotisch wirkenden Substanz (Osmagent) und im Falle der PPOP ein expandierendes Polymer-push-Kompartiment enthält.

Da semipermeable Membranen nur für das Medium, nicht aber für den Wirkstoff durchlässig sind, wird der aktive Inhaltsstoff durch mindestens ein Loch in der semipermeablen Membran freigesetzt.

Das wesentliche Ziel Osmotischer Pumpen, wie aus dem Stand der Technik bekannt, ist eine Wirkstofffreisetzung 0. Ordnung.

Im Gegensatz zu EOP und PPOP wurden auch Arzneiformen ohne semipermeable Membranen beschrieben, zum Beispiel: Controlled Porosity Osmotische Pumpen (CPOP).

CPOPs wurden auch entwickelt, um die aufwendige Produktion der oben beschriebenen Systeme, in die durch Bohrmaschinen oder Laser Freisetzungslöcher gebohrt werden müssen, zu ersetzen.

Diese CPOP Formulierungen besitzen eine wasserunlösliche Polymermembran, in die wasserlösliche Bestandteile eingearbeitet sind, welche nach Kontakt mit dem wässrigen Medium herausgelöst werden und dadurch eine mikroporöse Membran bilden, die nun für Medium und Wirkstoff permeabel ist.

Im Detail wird bei diesen Systemen der osmotische Tablettenkern mit einer unlöslichen Polymermembran umhüllt, in die wasserlösliche Substanzen eingearbeitet wurden. Nach dem Einbringen der Arzneiform in das Medium werden diese wasserlöslichen Substanzen herausgelöst.

Dabei entstehen Poren, durch die die Wirkstofffreisetzung erfolgt. Auch bei diesen Systemen handelt es sich um Tabletten, welche eine kontrollierte Freisetzung zeigen.

Dabei ist die Wirkstofffreisetzung im Besonderen abhängig von der Wasserlöslichkeit des Arzneistoffes und zeigt somit für pH-abhängig lösliche Wirkstoffe eine pH-abhängige Freisetzung.

Delayed Release Pelletformulierungen wurden beschrieben für osmagenthaltige Matrixpelletkerne, die mit einer semipermeablen Membran überzogen wurden. Diese Membran wird durch das Quellen des Kerns gedehnt, wodurch nach einer lag-time Poren entstehen, welche die Membran für Medium und Wirkstoff durchlässig machen und damit eine verzögerte Wirkstofffreisezung bewirken. Solche Delayed Release Formulierungen dienen der zielgenauen Wirkstofffreisetzung im GI-Trakt, einer Freisetzung nach chronopharmakologischen Aspekten oder werden bei nicht linearer Absorbtionskinetik eines Arzneistoffe verwendet.

Asymmetrische Membranen, die auf Tabletten und auch auf Pelletkerne aufgebracht werden können, bewirken eine verbesserte Freisetzung von schwach löslichen Wirkstoffen. Aber auch diese Formulierungen zeigen keine pH-unabhängige Wirkstofffreisetzung für pH-abhängig lösliche Substanzen. Eine pH-unabhängige Freisetzung wurde für solche Systeme beschrieben, wenn "pH-adjuster" zur Pufferung in der Kernformulierung eingearbeitet wurden. Solche Hilfsstoffe entweder Säuren oder Basen verändern den pH-Wert innerhalb der Formulierung soweit, dass die Wirkstofflöslichkeit, auch in pH-ungünstigen Medien, verbessert wird.

Weitere in der Literatur beschriebene Systeme zur pH-unabhängigen Wirkstofffreisetzung mittels "pH-adjuster" im Kern von Tabletten oder Pellets sind auch für nicht osmotisch wirkende Systeme beschrieben.

Multilayer Coatingkombinationen wurden beschrieben für die Kombination aus wasserlöslichen und wasserunlöslichen Polymerschichten, wobei die wasserlöslichen Polymere keine pH-abhängige Löslichkeit zeigen und damit auch keine Steuerung der Freisetzung pH-abhängig löslicher aktiver Inhaltsstoffe.

Des weiteren wurden Kombinationen aus wasserunlöslichen und pH-abhängig löslichen Polymerschichten und Polymermischungen beschrieben, wobei eine pH-unabhängige Wirkstofffreisetzung allein aufgrund unterschiedlicher Permeabilität des Polymerüberzuges erzielt wird. Dabei können Permeabilitäten des Polymerfilms genau eingestellt werden. Die pH-abhängig lösliche Polymerkomponente zeigt stets eine konträre Löslichkeit zum Wirkstoff. Schwache Wirkstoffbasen werden mit einem säureunlöslichen Polymer überzogen, wogegen bei schwachen Wirkstoffsäuren ein alkaliunlösliches Polymer als pH-abhängig lösliche Komponente verwendet wird. Es resultiert ein dünnerer oder poröserer Überzug in dem Medium, in dem die aktive Substanz schlechter löslich ist. Die Diffusionsbarriere im Medium mit schlechterer Wirkstofflöslichkeit wird somit verringert, woraus eine verbesserte Wirkstoffliberation resultiert.

### Offenbarung der Erfindung

Die vorliegende Erfindung bezieht sich auf eine feste Arzneimittelformulierung für eine retardierte pH-unabhängige Wirkstofffreisetzung enthaltend mindestens eine Schicht aus einem oder mehreren wasserunlöslichen Polymeren, mindestens eine Schicht aus einem oder mehreren pH-abhängigen wasserlöslichen Polymeren und einen wirkstoffhaltigen Kern, wobei der Kern einen Wirkstoff mit starker pH-abhängiger Löslichkeit im Wasser und mindestens einen Osmagenten enthält.

Nach einer bevorzugten Form der vorliegenden Erfindung enthält die Schicht aus einem oder mehreren wasserunlöslichen Polymeren porenbildende Substanzen, welche nach Kontakt mit dem wässrigen Medium herausgelöst werden und dadurch eine mikroporöse Membran bilden.

Nach einer weiteren Ausführungsform der Erfindung ist die Schicht aus einem oder mehreren pH-abhängigen wasserlöslichen Polymeren die äußere Schicht auf der festen Arzneimittelformulierung und die Schicht aus einem oder mehreren wasserunlöslichen Polymeren die innere.

Die vorliegende Erfindung bezieht sich weiter auf einen Matrixpellet für eine retardierte pH-unabhängige Wirkstofffreisetzung enthaltend mindestens eine innere Schicht aus einem oder mehreren wasserunlöslichen Polymeren, in denen porenbildende Substanzen enthalten sind, welche nach Kontakt mit dem wässrigen Medium herausgelöst werden und dadurch eine mikroporöse Membran bilden, mindestens eine äußere Schicht aus einem oder mehreren pH-abhängigen wasserlöslichen Polymeren, und einen wirkstoffhaltigen Kern, wobei der Kern Piperazinharnstoff und mindestens eine wasserlösliche ionische Substanz aus der Gruppe von Magnesiumchlorid, Magnesiumsulfat, Lithiumchlorid, Natriumchlorid, Kaliumchlorid, Lithiumsulfat, Natriumsulfat, Kaliumsulfat, Lithiumphosphat, Natriumphosphat, Kaliumphosphat, Ammoniumchlorid, Ammoniumsulfat, Ammoniumphosphat enthält.

Überraschender Weise reicht allein die Mischung von wasserunlöslichen mit pH-abhängig löslichen Polymeren bzw. der schichtweise Auftrag selbiger für eine retardierte pH-unabhängige Wirkstofffreisetzung nicht aus.

Dieses Phänomen konnte schon bei sehr geringen Auftragsmengen an pH-abhängig wasserlöslichem Polymer, zum Beispiel 2,5 und 5 % (w/w) bezogen auf Gesamtformulierungsmasse beobachtet werden.

Nur durch die Verwendung osmotisch wirksamer Substanzen gemäß der vorliegenden Erfindung wurde eine pH-unabhängige Wirkstofffreisetzung erreicht. Erst ein osmotisch aktiver Zusatz zur Kernformulierung mit hoher Wirkstoffbeladung zum Beispiel bis zum 90% w/w, bevorzugt bis zum 60%w/w, bezogen auf Kernformulierungsmasse bewirkt ein schnelles Eindringen von Medium in den Kern, gefolgt von der Bildung einer gesättigten Wirkstofflösung, die durch den osmotischen Druck getrieben, aus der festen Arzneimittelformulierung gedrückt wird.

Dadurch kann die Freisetzung der aktiven Substanz im Medium mit geringer Wirkstofflöslichkeit signifikant erhöht werden.

Erst hier wird die pH-abhängig lösliche Polymerschicht, durch das verstärkte Eindringen von Medium, soweit gedehnt, dass überhaupt ein signifikanter Austritt von Wirkstoff erreicht wird.

Nach der vorliegenden Erfindung ist eine pH-abhängig wasserlösliche Polymerschicht auch bei einem Kern mit Osmagent nötig.

Außerdem, nach einer weiteren bevorzugten Ausführungsform der Erfindung können porenbildende Substanzen ein Zusatz in der wasserunlöslichen Membran sein.

Durch eingearbeitete Porenbildner wird die Membran schnell durchlässig nicht nur für das Medium, sondern auch für den Wirkstoff. Die schnelle Durchlässigkeit der wasserunlöslichen Membran ist sehr wichtig insbesondere für Wirkstoffe mit einer sehr niedrigen Löslichkeit im Wasser.

In der festen Arzneimittelformulierung kann nach der vorliegenden Erfindung durch die Kombination von wasserunlöslichem Polymer mit oder ohne weitere wasserlösliche Substanzen zur Porenbildung und einem pH-abhängig wasserlöslichen Polymer nun eine pH-unabhängige Freisetzung der aktiven Substanz eingestellt werden.

Weiterhin ist die Freisetzung der aktiven Substanz aus der festen Arzneimittelformulierung nach der vorliegenden Erfindung nicht nur pH-unabhängig, sondern auch wesentlich erhöht im Vergleich zu den bekannten Arzneimittelformulierungen ohne Osmagent im Kern.

### Kurze Beschreibung der Zeichnungen

Die Erfindung wird im Folgenden anhand der Zeichnung näher erläutert.
Diese zeigt in:

Abbildung 1 eine bevorzugte Ausführungsform der festen Arzneimittelformulierung nach der vorliegenden Erfindung.

Abbildung 2 eine feste Arzneimittelformulierung von Abbildung 1 mit pH-abhängig wasserlöslicher Polymerschicht ohne Osmagent im Kern. Formulierungen mit pH-abhängig wasserlöslicher Polymerschicht ohne Osmagent im Kern zeigen eine sehr stark verminderte Wirkstofffreisetzung im Medium mit der eigentlich höchsten Wirkstofflöslichkeit.

Abbildungen 3a-3c zeigen Freisetzungsuntersuchungen von einer festen Arzneimittelformulierung von Abbildung 1 mit pH-abhängig wasserlöslicher Polymerschicht ohne Osmagent im Kern. Die Freisetzungsuntersuchungen wurden in einer USPXXV Basketapparatur bei 100 Umdrehungen pro Minute und einer Mediumtemperatur von 37 °C (+/- 0,5 °C) vorgenommen. Als Medien fanden 0,1 N HCl und Phosphatpuffer pH 6,8 Verwendung. Die Quantifizierung erfolgte mittels HPLC.

Abbildung 4 eine feste Arzneimittelformulierung von Abbildung 1 ohne pH-abhängig wasserlöslicher Polymerschicht mit Osmagent im Kern.

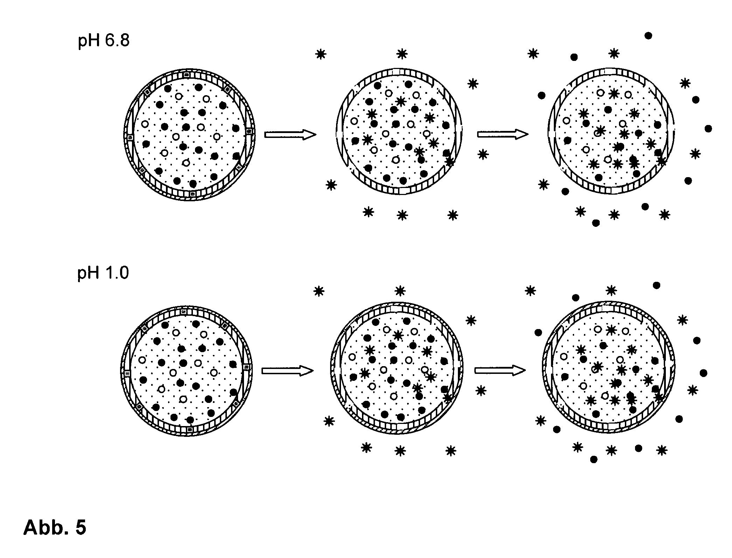

Abbildung 5 eine feste Arzneimittelformulierung von Abbildung 1 mit pH-abhängig wasserlöslicher Polymerschicht und Osmagent im Kern. Durch das Einbringen osmotisch wirksamer Substanzen wurde eine pH-unabhängige Wirkstofffreisetzung erreicht.

Abbildungen 6a-6c und 7a-7c zeigen Freisetzungsuntersuchungen der festen Arzneimittelformulierung von Abbildung 1 mit pH-abhängig wasserlöslicher Polymerschicht mit Osmagent im Kern (Abbildung 6a-6c für Beispiele 2 und Abbildung 7a-7c für Beispiele 3). Die Freisetzungsuntersuchungen wurden in einer USPXXV Basketapparatur bei 100 Umdrehungen pro Minute und einer Mediumtemperatur von 37 °C (+/- 0,5 °C)vorgenommen. Als Medien fanden 0,1 N HCl und Phosphatpuffer pH 6,8 Verwendung. Die Quantifizierung erfolgte mittels HPLC.

### Ausführungsform(en) der Erfindung

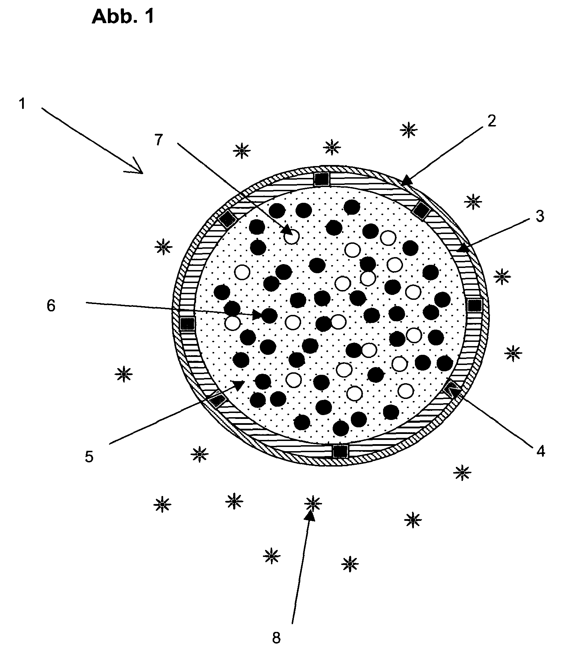

Die erfindungsgemäße feste Arzneimittelformulierung 1 (Abb. 1) enthält mindestens eine Schicht 3, aus einem oder mehreren wasserunlöslichen Polymeren, mindestens eine Schicht 2 aus einem oder mehreren pH-abhängig wasserlöslichen Polymeren.

Der Formulierungskern 5 gemäß der Erfindung ist mit einem starken pH-abhängigen wasserlöslichen Wirkstoff 6 und mindestens einem Osmagent 7 beladen.

Nach einer bevorzugten Ausführungsform der Erfindung enthält die Schicht 3 aus einem oder mehreren wasserunlöslichen Polymeren porenbildende Substanzen 4, welche nach Kontakt mit dem wässrigen Medium 8 herausgelöst werden und dadurch eine mikroporöse Membran bilden.

Die eine oder mehrere porenbildenden Substanzen 4 können wasserlösliche Polymere oder andere wasserlösliche Zusätze wie Salze oder Zuckern sein.

Die eine oder mehrere porenbildenden Substanzen 4 können aus der Gruppe enthaltend zum Beispiel Polyvinylpyrrolidon (PVP), Crospovidon (Cross-linked-N-vinyl-2-pyrolidone, cl-PVP), Hydroxypropylmethylcellulose (HPMC), Polyethylenglykol (PEG), Hydroxypropylcellulose (HPC) und deren Gemischen ausgewählt sein.

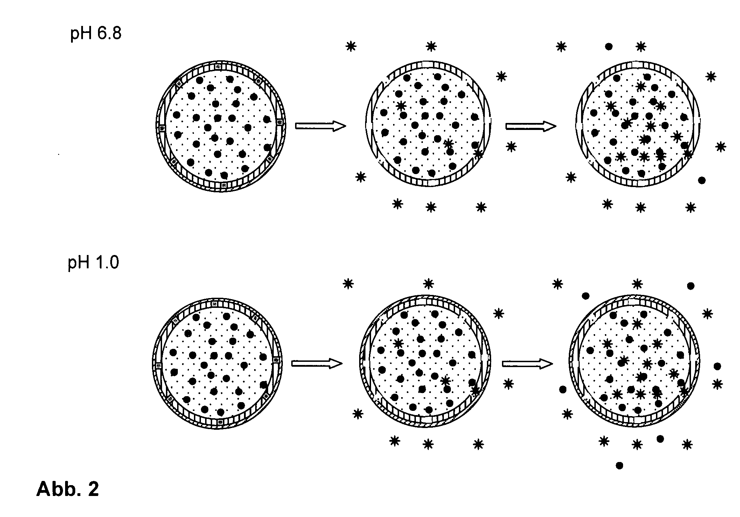

Formulierungen aus einem wirkstoffhaltigen Kern ohne Osmagent und aus zwei Schichten Polymer (Abb. 2), wobei die innere Schicht aus einem wasserunlöslichen und die äußere Schicht aus einem pH-abhängig wasserlöslichen Polymer bestand, zeigten noch immer eine starke pH-abhängige Freisetzung der aktiven Substanz.

Besonders auffällig war die sehr stark verminderte Wirkstofffreisetzung im Medium mit der eigentlich höchsten Wirkstofflöslichkeit.

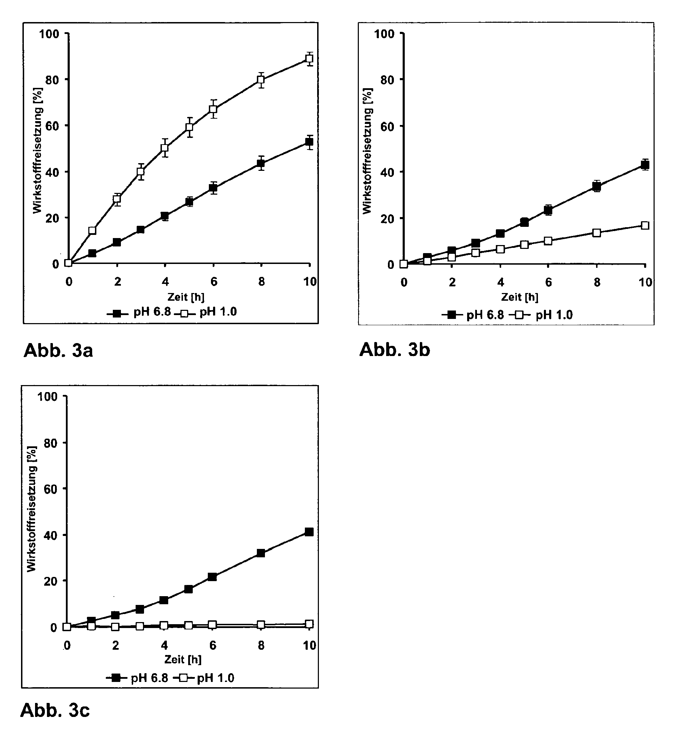

Zum Beispiel ein Piperazinharnstoff enthaltender Kern ohne Osmagent nach Abbildung 2 mit höherer Löslichkeit bei sauren pH-Werten (pH < 4) konnte eine effektive Wirkstofffreisetzung nicht erreichen (Abb. 3a-c). Die Wirkstofffreisetzung im Medium mit pH 1 von Piperazinharnstoff war geringer als erwartet.

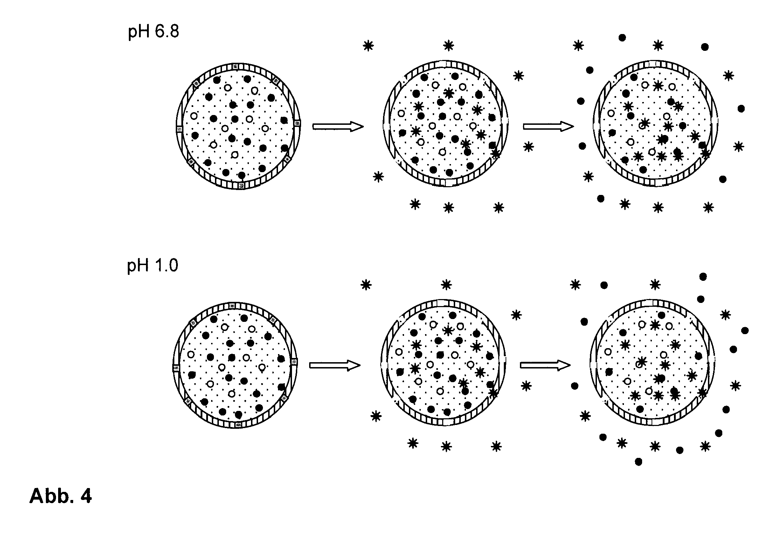

Auch bei einer Formulierung ohne pH-abhängig wasserlöslichem Polymerfilm (Abb. 4) wird eine pH-unabhängig Wirkstofffreisetzung nicht erreicht.

Durch die erfindungsgemäße feste Arzneimittelformulierung können die effektiven retardierten pH-unabhängigen Wirkstofffreisetzungen 0. oder 1. Ordnung leicht erreicht werden.

Im Folgenden ist ein Herstellungsbeispiel der festen Arzneimittelformulierung für eine retardierte pH-unabhängige Wirkstofffreisetzung nach der vorliegenden Erfindung beschrieben.

Eine trockene Pulvermischung wurde durch das Einbringen der gesiebten Bestandteile in ein Müllerfass mit anschließendem Mischen in einem Turbulamischer hergestellt.

Die trockene Pulvermischung wurde anschließend in einem Schnellmischer befeuchtet, wobei durch Vorversuche die zum Extrudieren und Sphäronisieren nötige Binderlösungsmenge bestimmt wurde. Das entstandene feuchte Granulat wurde danach im Extruder extrudiert und im Spheronizer verrundet.

Die erzeugten Pellets nach einer bevorzugten Ausführungsform der Erfindung wurden dann in der Wirbelschicht (GPCG1 der Fa. Glatt) getrocknet.

Nach der Siebung wurde die Pelletfraktion von 0,8 mm bis 1,25 mm Durchmesser für die weitere Herstellung verwendet.

Die Polymerdispersionen wurden im Wirbelschichtgranulator mit WursterEinsatz aufgetragen. Wobei nach dem Auftrag der ersten Schicht eine kurze Trocknungspause dem Auftrag der zweiten Schicht vorangeht.

Die Formulierungsschicht aus einem oder mehreren wasserunlöslichen Polymeren (Subcoatingformulierung) ist zum Beispiel von 1 % bis 40% w/w, bevorzugt von 1 % bis 10% w/w, bevorzugt von 2% bis 5% w/w bezogen auf Gesamtformulierungsmasse. Die wasserunlöslichen Polymere können aus der Gruppe enthaltend Polyvinylacetat; Alkylcellulosen, Acrylat-Methacrylat-Copolymere, Vinylacetat-Methacrylat-Copolymere und -Acrylat-Copolymere; Ethylcellulose, Ethylacrylat-Methylmethacrylat-Copolymer und Ethylacrylat-Methylacrylat-Trimethylammoniummethylmethacrylatchlorid-Terpolymer und deren Gemischen ausgewählt sein.

Nach einer bevorzugten Ausführungsform der Erfindung wurden porenbildende Substanzen in der Formulierungsschicht aus einem oder mehreren wasserunlöslichen Polymeren (Subcoatingformulierung) verwendet. Die eine oder mehrere porenbildenden Substanzen können wasserlösliche Polymere oder andere wasserlösliche Zusätze wie Salze oder Zucker sein. Nach einer bevorzugten Ausführungsform der Erfindung, die eine oder mehrere porenbildenden Substanzen können aus der Gruppe enthaltend zum Beispiel Polyvinylpyrrolidon (PVP), Crospovidon (Cross-linked-N-vinyl-2-pyrolidone, cl-PVP), Hydroxypropylmethylcellulose (HPMC), polyethylenglykol (PEG), Hydroxypropylcellulose (HPC) und deren Gemischen ausgewählt sein.

Der Formulierungsschicht aus einem oder mehreren pH-abhängigen wasserlöslichen Polymeren (Topcoatingformulierung) ist zum Beispiel von 1 % bis 40% w/w, bevorzugt von 1 % bis 10% w/w, bevorzugt von 2% bis 5% w/w bezogen auf Gesamtformulierungsmasse.

Die säureunlöslichen Polymere können aus der Gruppe enthaltend Acrylat-Methacrylsäure-Copolymere, Carboxyalkylcellulosen, Celluloseacetatphthalate, Celluloseacetatsuccinate, Celluloseacetatrimelliate, Hydroxyalkylcellulosephthalate, Hydroxyalkylcelluloseacetatsuccinate, Vinylacetatphthalate, Vinylacetatsuccinat; Ethylacryl-Methacrylsäure-Copolymer, Methylmethacrylat-Methacrylsäure-Copolymer, Methylmethacrylat-Methylacrylat-Methacrylsäure-Copolymer, Carboxymethylcellulose, Celluloseacetatphthalat; Hydroxypropylmethylcellulosephthalat, Hydroxypropylmethylcelluloseacetatphthalat, Hydroxypropylmethylcelluloseacetatsuccinat, Polyvinylacetatphthalat, Schellack und deren Gemischen ausgewählt sein.

Als alkaliunlösliche Polymere können Acrylat-Methacrylat-Copolymere, basische natürliche Polysaccaride, Dimethylaminoethylmethacrylat-Methylmethacrylat-Butylmethacrylat-Terpolymer, Chitosan und deren Gemischen verwendet werden.

Als osmotisch aktive Substanzen (Osmagentien) zur gezielten pH-unabhängigen Wirkstofffreisetzung können wasserlösliche ionische oder nichtionische Substanzen und hydrophile Polymere, allein oder als Mischung, verwendet werden.

Die wasserlösliche ionische Substanz kann aus der Gruppe enthaltend Magnesiumchlorid, Magnesiumsulfat, Lithiumchlorid, Natriumchlorid, Kaliumchlorid, Lithiumsulfat, Natriumsulfat, Kaliumsulfat, Lithiumphosphat, Natriumphosphat, Kaliumphosphat, Natriumcarbonat, Ammoniumchlorid, Ammoniumsulfat, Ammoniumphosphat allein oder als Mischung ausgewählt sein.

Die wasserlösliche ionische osmotische Substanz kann von 2% bis 50% w/w bezogen auf Gesamtkernmasse und insbesondere von 2% bis 20% w/w bezogen auf Gesamtkernmasse im Kern enthaltend sein.

Eine wasserlösliche nichtionische Substanz kann aus der Gruppe enthaltend zum Beispiel Saccharose, Mannitol, Lactose, Dextrose, Sorbitol, allein oder als Mischung ausgewählt sein.

Die wasserlösliche nichtionische osmotische Substanz kann von 2% bis 50% w/w bezogen auf Gesamtkernmasse und insbesondere von 10% bis 40% w/w bezogen auf Gesamtkernmasse im Kern enthaltend sein.

Die Hydrophile Polymere können aus der Gruppe enthaltend Hydroxypropylmethylcellulose (HPMC), Hydroxypropylcellulose (HPC), Xanthan Gum, Alginat, Natrium Carboxylmetylcellulose, Polyvinylpyrrolidon (PVP), CI-Polyvinylpyrrolidon (CI-PVP), Polyethylenoxid, Carbopole, Polyacrylamine, Arabisches Gummi und deren Gemischen ausgewählt sein.

Nach der vorliegenden Erfindung sind wasserlöslichen ionischen Substanzen bevorzugt verwendet, die mit geringeren Mengen eine hohe osmotische Wirkung erreichen.

Als zusätzliches Formulierungsmittel zur Beeinflussung der mechanischen Festigkeit der Arzneiform können Cellulose oder Cellulosederivate verwendet werden. Besonders vorteilhaft ist mikrokristalline Cellulose.

### Beispiele

Beispiel 1: Herstellung überzogener Matrixpellets mit pH-abhängiger wasserlöslicher Polymerschicht ohne Osmagent im Kern (Abb. 3a-c; Stand der Technik)

**Kernformulierung (%w/w):**

| | |
|---|---|
| Wirkstoff (Piperazinharnstoff) | 60% |
| Mikrokristalline Cellulose | 40% |

**Formulierungsschicht aus einem oder mehreren wasserunlöslichen Polymeren (Subcoatinqformulierung) (%w/w):**

| | |
|---|---|
| Polyvinylacetat | 70% |
| Polyvinylpyrrolidon | 30% |
| Coatinglevel der Subcoatingformulierung: | 5% w/w bezogen auf Gesamtpelletmasse. |

**Formulierungsschicht aus einem oder mehreren pH-abhängigen wasserlöslichen Polymeren (Topcoatingformulierung) (%w/w):**

| | |
|---|---|
| Methacrylsäure-Ethylacrylat-Copolymer | 100% |
| Coatinglevel der Topcoatingformulierung (%w/w): | 0% (Abb. 3a); 2,5% (Abb. 3b); 5% (Abb. 3c) w/w_bezogen auf Gesamtpelletmasse. |

Mikrokristalline Cellulose und Wirkstoff werden gesiebt und im Turbula-Mischer für 20 Minuten gemischt.

Die trockene Pulvermischung wird im Schnellmischer mit der nötigen Menge Binderlösung (Wasser) versetzt. Das erhaltenen feuchte Granulat wird nachfolgend im Extruder durch ein 1 mm Sieb extrudiert.

Das erzeugte Extrudat wird portionsweise im Spheronizer bei 400 UpM verrundet. Im Anschluss erfolgt die Trocknung der Pellets im Wirbelschichtgranulator GPCG1 bei 60°C.

Nach der Siebung wurde die Pelletfraktion von 0,8 mm bis 1,25 mm Durchmesser für die weitere Herstellung verwendet.

Die Matrixpelletkerne werden im Wirbelschichtgranulator GPCG1 mit Wurstereinsatz 10 Minuten bei 50°C temperiert. Danach wird eine 15% (w/w) Polyvinylacetatdispersion, die den wasserlöslichen Porenbildner Polyvinylpyrrolidon enthält, bei 50°C Zulufttemperatur aufgetragen.

Nach 10 min Zwischentrocknen wird die pH-abhängig lösliche Methacrylsäure-Ethylacrylat-Copolymer (15% w/w) bei 50°C Zulufttemperatur aufgesprüht.

Nach erfolgtem Polymerauftrag werden die überzogenen Matrixpellets 24h bei 40 °C getempert.

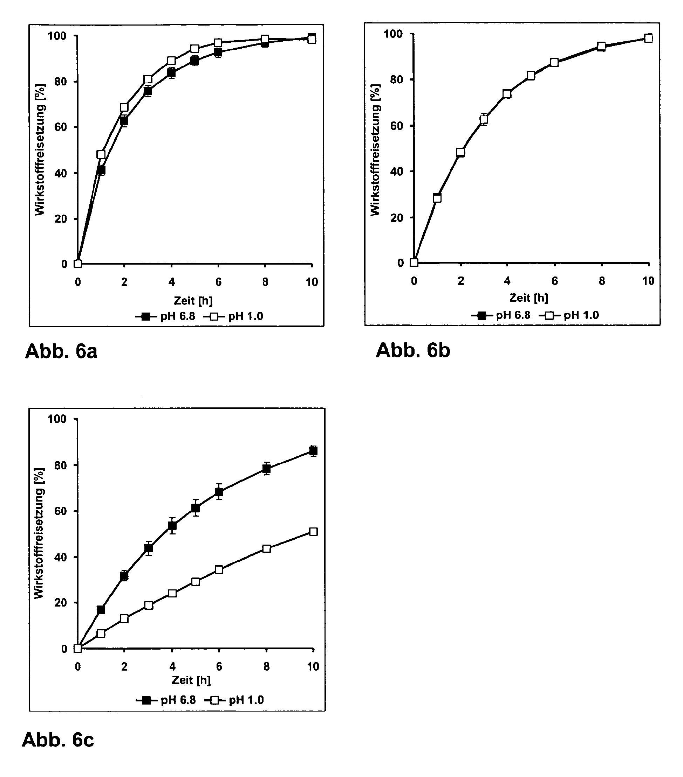

Beispiel 2: Herstellung überzogener Matrixpellets mit pH-abhängiger wasserlöslicher Polymerschicht mit Osmagent (KCI) im Kern (Abb. 6a-c).

**Kernformulierung (%w/w):**

| | |
|---|---|
| Wirkstoff (Piperazinharnstoff) | 60% |
| Osmotisch wirksame Substanz (KCI) | 15% |
| Mikrokristalline Cellulose | 25% |

**Formulierunqsschicht aus einem oder mehreren wasserunlöslichen Polymeren (Subcoatingformulierunq) (%w/w):**

| | |
|---|---|
| Polyvinylacetat | 70% |
| Polyvinylpyrrolidon | 30% |
| Coatinglevel der Subcoatingformulierung: | 5%w/w bezogen auf Gesamtpelletmasse. |

**Formulierungsschicht aus einem oder mehreren pH-abhängigen wasserlöslichen Polymeren (Topcoatingformulierung) (%w/w):**

| | |
|---|---|
| Methacrylsäure-Ethylacrylat-Copolymer | 100% |
| Coatinglevel der Topcoatingformulierung (%w/w): | 0% (Abb. 6a); 2,5% (Abb. 6b); 5%(Abb. 6c) bezogen auf Gesamtpelletmasse. |

Mikrokristalline Cellulose und Wirkstoff werden gesiebt und im Turbula-Mischer für 10 Minuten gemischt. Gesiebtes Kaliumchlorid wird zugesetzt und erneut 10 Minuten im Turbula-Mischer gemischt.

Die trockenen Pulvermischung wird im Schnellmischer mit der nötigen Menge Binderlösung (Wasser) versetzt. Das erhaltenen feuchte Granulat wird nachfolgend im Extruder durch ein 1 mm Sieb extrudiert.

Das erzeugte Extrudat wird portionsweise im Spheronizer bei 400 UpM verrundet. Im Anschluß erfolgt die Trocknung der Pellets im Wirbelschichtgranulator GPCG1 bei 60°C.

Nach der Siebung wurde die Pelletfraktion von 0,8 mm bis 1,25 mm Durchmesser für die weitere Herstellung verwendet.

Die Matrixpelletkerne werden im Wirbelschichtgranulator GPCG1 mit Wurstereinsatz 10 Minuten bei 50°C temperiert. Danach wird eine 15% (w/w) Polyvinylacetatdispersion, die den wasserlöslichen Porenbildner Polyvinylpyrrolidon enthält, bei 50°C Zulufttemperatur aufgetragen.

Nach 10 min Zwischentrocknen wird die pH-abhängig lösliche Methacrylsäure-Ethylacrylat-Copolymer (15% w/w) bei 50°C Zulufttemperatur aufgesprüht.

Nach erfolgtem Polymerauftrag werden die überzogenen Matrixpellets 24h bei 40 °C getempert.

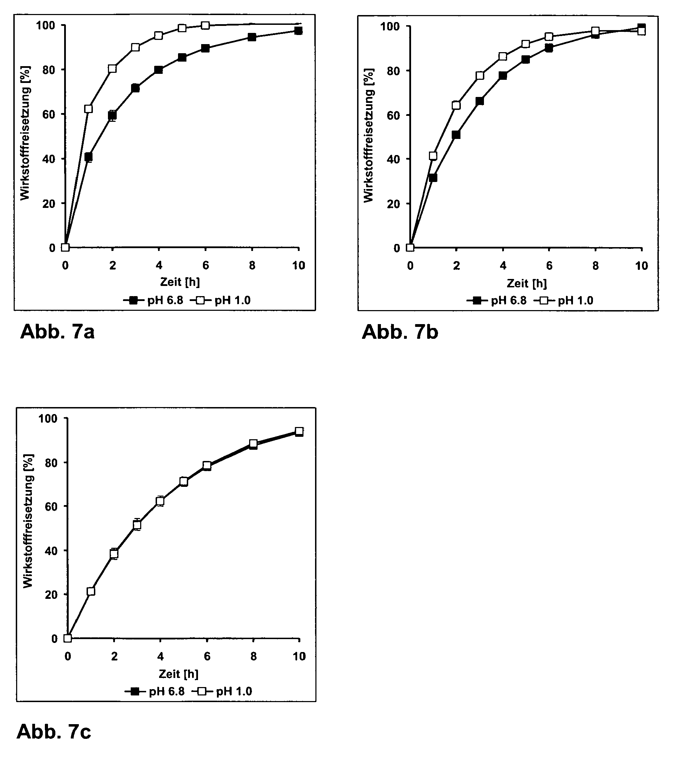

Beispiel 3: Herstellung überzogener Matrixpellets mit pH-abhängiger wasserlöslicher Polymerschicht mit Osmagent (NaCl) im Kern (Abb. 7a-c).

**Kernformulierung (%w/w):**

| | |
|---|---|
| Wirkstoff (Piperazinharnstoff) | 60% |
| Osmotisch wirksame Substanz (NaCl) | 15% |
| Mikrokristalline Cellulose | 25% |

**Formulierungsschicht aus einem oder mehreren wasserunlöslichen Polymeren (Subcoatingformulierung) (%w/w):**

| | |
|---|---|
| Polyvinylacetat | 70% |
| Polyvinylpyrrolidon | 30% |
| Coatinglevel der Subcoatingformulierung: | 5%w/w bezogen auf Gesamtpelletmasse. |

**Formulierungsschicht aus einem oder mehreren pH-abhängigen wasserlöslichen Polymeren (Topcoatingformulierung):**

| | |
|---|---|
| Methacrylsäure-Ethylacrylat-Copolymer | 100% |
| Coatinglevel der Topcoatingformulierung (%w/w): | 0% (Abb. 7a); 3% (Abb. 7b); 4% (Abb. 7c) bezogen auf Gesamtpelletmasse. |

Mikrokristalline Cellulose und Wirkstoff werden gesiebt und im Turbula-Mischer für 10 Minuten gemischt. Gesiebtes Natriumchlorid wird zugesetzt und erneut 10 Minuten im Turbula-Mischer gemischt.

Die trockene Pulvermischung wird im Schnellmischer mit der nötigen Menge Binderlösung (Wasser) versetzt. Das erhaltenen feuchte Granulat wird nachfolgend im Extruder durch ein 1 mm Sieb extrudiert.

Das erzeugte Extrudat wird portionsweise im Spheronizer bei 400 UpM verrundet. Im Anschluss erfolgt die Trocknung der Pellets im Wirbelschichtgranulator GPCG1 bei 60°C.

Nach der Siebung wurde die Pelletfraktion von 0,8 mm bis 1,25 mm Durchmesser für die weitere Herstellung verwendet.

Die Matrixpelletkerne werden im Wirbelschichtgranulator GPCG1 mit Wurstereinsatz 10 Minuten bei 50°C temperiert. Danach wird eine 15% (w/w) Polyvinylacetatdispersion, die den wasserlöslichen Porenbildner Polyvinylpyrrolidon enthält, bei 50°C Zulufttemperatur aufgetragen.

Nach 10 min Zwischentrocknen wird die pH-abhängig lösliche Methacrylsäure-Ethylacrylat-Copolymer (15% w/w) bei 50°C Zulufttemperatur aufgesprüht.

Nach erfolgtem Polymerauftrag werden die überzogenen Matrixpellets 24h bei 40 °C getempert.

## Patentansprüche

1. Feste Arzneimittelformulierung für eine retardierte pH-unabhängige Wirkstofffreisetzung enthaltend
mindestens eine Schicht aus einem oder mehreren wasserunlöslichen Polymeren
mindestens eine Schicht aus einem oder mehreren pH-abhängigen wasserlöslichen Polymeren, und
einen wirkstoffhaltigen Kern, wobei
der Kern einen Wirkstoff mit starker pH-abhängiger Wasserlöslichkeit und mindestens einen Osmagenten enthält.

2. Feste Arzneimittelformulierung nach Anspruch 1, wobei die Schicht aus einem oder mehreren pH-abhängigen wasserlöslichen Polymeren die äußere Schicht auf der festen Arzneimittelformulierung ist und die Schicht aus einem oder mehreren wasserunlöslichen Polymeren die innere.

3. Feste Arzneimittelformulierung nach Anspruch 1 und 2, wobei die Schicht aus einem oder mehreren wasserunlöslichen Polymeren von 1 % bis 40% w/w bezogen auf Gesamtformulierungsmasse ist.

4. Feste Arzneimittelformulierung nach Anspruch 1-3, wobei die Schicht aus einem oder mehreren wasserunlöslichen Polymeren von 1 % bis 10% w/w bezogen auf Gesamtformulierungsmasse ist.

5. Feste Arzneimittelformulierung nach Anspruch 1-4, wobei die Schicht aus einem oder mehreren wasserunlöslichen Polymeren von 2% bis 5% w/w bezogen auf Gesamtformulierungsmasse ist.

6. Feste Arzneimittelformulierung nach Anspruch 1-5, wobei ein oder mehrere wasserunlösliche Polymere aus der Gruppe enthaltend Polyvinylacetat, Alkylcellulosen, Acrylat-Methacrylat-Copolymere, Vinylacetat-Methacrylat-Copolymere und Acrylat-Copolymere, Ethylcellulose, Ethylacrylat-Methylmethacrylat-Copolymer und Ethylacrylat-Methylacrylat-Trimethylammoniummethylmethacrylatchlorid-Terpolymer und deren Gemischen ausgewählt sind.

7. Feste Arzneimittelformulierung nach Anspruch 1-6, wobei die Schicht aus einem oder mehreren wasserunlöslichen Polymeren eine oder mehrere porenbildende Substanzen enthält.

8. Feste Arzneimittelformulierung nach Anspruch 1-7, wobei die porenbildende Substanz aus der Gruppe enthaltend Polyvinylpyrrolidon, Crospovidon, Hydroxypropylmethylcellulose, Polyethylenglykol, Hydroxypropylcellulose und deren Gemischen ausgewählt ist.

9. Feste Arzneimittelformulierung nach Anspruch 1-8, wobei die Schicht aus einem oder mehreren pH-abhängigen wasserlöslichen Polymeren von 1 % bis 40% w/w bezogen auf Gesamtformulierungsmasse ist.

10. Feste Arzneimittelformulierung nach Anspruch 1-9, wobei die Schicht aus einem oder mehreren pH-abhängigen wasserlöslichen Polymeren von 1 % bis 10% w/w bezogen auf Gesamtformulierungsmasse ist.

11. Feste Arzneimittelformulierung nach Anspruch 1-10, wobei die Schicht aus einem oder mehreren pH-abhängigen wasserlöslichen Polymeren von 2% bis 5% w/w bezogen auf Gesamtformulierungsmasse ist.

12. Feste Arzneimittelformulierung nach Anspruch 1-11, wobei ein oder mehrere säureunlösliche Polymere ausgewählt aus der Gruppe enthaltend Acrylat-Methacrylsäure-Copolymere, Carboxyalkylcellulosen, Celluloseacetatphthalate, Celluloseacetatsuccinate, Celluloseacetatrimelliate, Hydroxyalkylcellulosephthalate, Hydroxyalkylcelluloseacetatsuccinate, Vinylacetatphthalate, Vinylacetatsuccinat; Ethylacryl-Methacrylsäure-Copolymer, Methylmethacrylat-Methacrylsäure-Copolymer, Methylmethacrylat-Methylacrylat-Methacrylsäure-Copolymer, Carboxymethylcellulose, Celluloseacetatphthalat; Hydroxypropylmethylcellulosephthalat, Hydroxypropylmethylcelluloseacetatphthalat, Hydroxypropylmethylcelluloseacetatsuccinat, Polyvinylacetatphthalat, Schellack und deren Gemischen sind.

13. Feste Arzneimittelformulierung nach Anspruch 1-12, wobei ein oder mehrere alkaliunlösliche Polymere ausgewählt aus der Gruppe enthaltend Acrylat-Methacrylat-Copolymere, basische natürliche Polysaccaride, Dimethylaminoethylmethacrylat-Methylmethacrylat-Butylmethacrylat-Terpolymer, Chitosan und deren Gemischen sind.

14. Feste Arzneimittelformulierung nach Anspruch 1-13, wobei der Kern mit bis zu 90% w/w bezogen auf Kernformulierungsmasse eines Wirkstoffs mit starker pH-abhängiger Wasserlöslichkeit beladen ist.

15. Feste Arzneimittelformulierung nach Anspruch 1-14, wobei der Kern mit bis zu 60% w/w bezogen auf Kernformulierungsmasse eines Wirkstoffs mit starker pH-abhängiger Wasserlöslichkeit beladen ist.

16. Feste Arzneimittelformulierung nach Anspruch 1-15, wobei der Kern mit Piperazinharnstoff beladen ist.

17. Feste Arzneimittelformulierung nach Anspruch 1-16, wobei der Osmagent im Kern aus der Gruppe enthaltend wasserlösliche ionische Substanzen, wasserlösliche nichtionische Substanzen, hydrophile Polymere und deren Gemischen ausgewählt ist.

18. Feste Arzneimittelformulierung nach Anspruch 1-17, wobei der Osmagent im Kern aus der Gruppe enthaltend Magnesiumchlorid, Magnesiumsulfat, Lithiumchlorid, Natriumchlorid, Kaliumchlorid, Lithiumsulfat, Natriumsulfat, Kaliumsulfat, Lithiumphosphat, Natriumphosphat, Kaliumphosphat, Ammoniumchlorid, Ammoniumsulfat, Ammoniumphosphat, Saccharose, Mannitol, Lactose, Dextrose, Sorbitol, Hydroxypropylmethylcellulose, Hydroxypropylcellulose, Xanthan Gum, Alginat, Natrium Carboxylmetylcellulose, Polyvinylpyrrolidon, CI-Polyvinylpyrrolidon, Polyethylenoxid, Carbopole, Polyacrylamine, Arabisches Polymere und deren Gemischen ausgewählt ist.

19. Feste Arzneimittelformulierung nach Anspruch 1-18, die ein Matrixpellet ist.

20. Feste Arzneimittelformulierung nach Anspruch 1-19, wobei der Kern einen Durchmesser von 0,8 mm bis 1,25 mm hat.
